# EUROPEAN PATENT APPLICATION

(11) **EP 4 521 053 A2**
(43) Date of publication of application: **12.03.2025**
(21) Application number: 25154512.5
(22) Date of filing: 23.12.2020
(51) Int. Cl.: F28D 15/00

(54) **HEATER AND COOLER SYSTEM WITH DISPOSABLE HEAT TRANSFER FLUID MODULE**

(30) Priority: 31.12.2019 US 201962955816 P
(62) Divisional of application: 20830347.9
(71) Applicant: LivaNova Deutschland GmbH, 80939 Munich (DE)
(72) Inventor: BONCZAR, Michael, 84539 Ampfing (DE); WOLFGRAMM, Olivier, 81479 München (DE)
(74) Representative: Buzzi, Notaro & Antonielli d'Oulx S.p.A.

(57) **Abstract**

A system for heating/cooling a target unit. The system including a heater/cooler unit and a heat transfer fluid module. The heater/cooler unit including a heater/cooler that includes a heater/cooler element and a heater/cooler pump, and a heat exchanger that includes a heat exchange element. Where, the heater/cooler pump pumps a first fluid through the heater/cooler element and the heat exchange element and back to the heater/cooler pump. The heat transfer fluid module including a fluid reservoir with a second fluid that is pumped to and through the heat exchanger to transfer heating/cooling between the first fluid and the second fluid and pumped to and through the target unit to transfer heating/cooling between the second fluid and the target unit.

## Description

### TECHNICAL FIELD

The present disclosure relates to a system for heating and/or cooling (heating/cooling) a target unit. More specifically, the disclosure relates to a system for heating/cooling a patient or organs or other fluids, like blood, either directly or through a secondary fluid circuit, e.g., a heat exchanger, in an oxygenator of a heart- lung machine during extracorporeal blood circulation. Background documents in this field include, i.a., WO 2018/002622 A1, WO 2019/016542 A1, and WO 2019/068342 A1.

### BACKGROUND

Oxygenators are devices used for extracorporeal oxygenation of blood. Often, oxygenators are used in heart-lung machines or extracorporeal membrane oxygenation (ECMO) devices, which include membrane oxygenators that can avoid embolisms to a large extent. With the aid of gas mixers and flow meters the transfer of oxygen and carbon dioxide is reliably controlled.

In an oxygenator, a patient's blood is warmed or cooled and oxygenated. The oxygenator optionally includes a heat exchanger for warming or cooling the blood. In the oxygenator, a heat exchanging medium flows through the heat exchanger and transfers a heat quantity to the blood for warming the blood or absorbs a heat quantity from the blood for cooling the blood. The heat exchanging medium is usually supplied to the heat exchanger by a pump unit and, after heat exchange with the blood has taken place, the heat exchanging medium is discharged from the heat exchanger by the same pump unit or another pump unit. The heat exchanging medium, e.g. water, glycol, or combinations of these or other fluids, is previously heated or cooled in a heater/cooler before it is conducted to the heat exchanger. Due to its size and complex structure, the heater/cooler is separate from the heart-lung machine or ECMO.

In some situations, the heat exchange medium of the heater/cooler and system configurations rely on reusing the heater/cooler fluids or media, allow for the unintended reuse of heater/cooler fluids or media, or depend on a user or operator to follow proper cleaning and maintenance instructions or protocols. If the operator does not follow proper instructions or protocols, these situations may create the potential for the build-up of contaminates and potentially harmful microorganisms in the heater/cooler fluids or media. Also, the heater/cooler may be over powered for most applications and present power consumption and power supply compatibility issues. In addition, due to its large size, the heater/cooler may have limited usability and may not be transportable. Also, due to its size to power ratio the heater/cooler may not be suited for intensive care unit (ICU) applications.

### SUMMARY

As recited in examples, example 1 is a system for heating/cooling a target unit. The system includes a heater/cooler unit and a heat transfer fluid module. The heater/cooler unit includes a heater/cooler that includes a heater/cooler element and a heater/cooler pump, and a heat exchanger that includes a heat exchange element. The heater/cooler pump pumps a first fluid through the heater/cooler element and the heat exchange element and back to the heater/cooler pump. The heat transfer fluid module includes a fluid reservoir with a second fluid that is pumped to and through the heat exchanger to transfer heating/cooling between the first fluid and the second fluid and pumped to and through the target unit to transfer heating/cooling between the second fluid and the target unit.

Example 2 is the system of Example 1, wherein the heater/cooler pump, the heater/cooler element, and the heat exchange element are a closed circuit containing the first fluid.

Example 3 is the system of Examples 2 or 3, wherein the heater/cooler pump, the heater/cooler element, and the heat exchange element are a hermetically sealed closed circuit containing the first fluid and configured to prevent contamination of the second fluid and an operating room.

Example 4 is the system of any of Examples 1-3, wherein the heat transfer fluid module is a disposable heat transfer fluid module.

Example 5 is the system of any of Examples 1-4, wherein the heat transfer fluid module includes the fluid reservoir and a heat transfer fluid pump configured to pump the second fluid from the fluid reservoir to the heat exchanger and the target unit and back to the fluid reservoir.

Example 6 is the system of any of Examples 1-5, wherein the heat transfer fluid pump is a reversible pump configured to drain the second fluid from the heat exchanger and return the second fluid to the fluid reservoir.

Example 7 is the system of any of Examples 1-6, wherein the heat exchanger includes a heat transfer fluid pump configured to pump the second fluid from the fluid reservoir to the heat exchanger and the target unit and back to the fluid reservoir.

Example 8 is the system of any of Examples 1-7, wherein the heat transfer fluid pump is a reversible pump configured to drain the second fluid from the heat exchanger and return the second fluid to the fluid reservoir.

Example 9 is the system of any of Examples 1-8, comprising an integrated thermal disinfection system around the heat exchanger to provide heat to disinfect the heat exchanger.

Example 10 is the system of any of Examples 1-9, wherein the heat exchanger is emptied of the second fluid and hot disinfected by the thermal disinfection system at a higher temperature for a period to sterilize the heat exchanger and prevent bacterial growth.

Example 11 is a system for heating/cooling a target unit. The system including a heater/cooler unit and a heat transfer fluid module. The heater/cooler unit configured to regulate temperature of a first fluid and pump the first fluid through a heat exchanger in a closed circuit that prevents contamination due to the first fluid. The heat transfer fluid module including a fluid reservoir and a heat transfer fluid pump that pumps a second fluid from the fluid reservoir to the heat exchanger and the target unit and back to the fluid reservoir to regulate temperature of the second fluid and the target unit.

Example 12 is the system of Example 11, wherein the heat transfer fluid module includes the fluid reservoir, the heat transfer fluid pump, and a pass though tube configured to pass the second fluid from the heat exchanger to the target unit.

Example 13 is the system of Example 11 or 12, wherein the heat transfer fluid pump is a reversible pump configured to drain the second fluid from the heat exchanger and return the second fluid to the fluid reservoir.

Example 14 is the system of any of Examples 11-13, wherein the heat transfer fluid pump is a reversible pump including a flexible impeller having flexible blades and configured to bend the flexible blades counter to a direction of the impeller inside the heat transfer fluid pump.

Example 15 is the system of any of Examples 11-14, wherein the heat transfer fluid module is a disposable heat transfer fluid module.

Example 16 is the system of any of Examples 11-15, comprising an integrated thermal disinfection system surrounding at least some of the heat exchanger and configured to thermally disinfect the heat exchanger at a higher temperature for a specified time to sterilize the heat exchanger.

Example 17 is the system of any of Examples 11-16, wherein the heater/cooler unit includes a drive motor configured to drive the heat transfer fluid pump.

Example 18 is a method of heating/cooling a target fluid in a target unit using a heater/cooler unit and a heat transfer fluid module. The heater/cooler unit including a heater/cooler pump, a heater/cooler element, and a heat exchanger and the heat transfer fluid module including a fluid reservoir. The method including: pumping a first fluid, using the heater/cooler pump, through the heater/cooler element and the heat exchanger and back to the heater/cooler pump in a closed circuit; heating/cooling the first fluid with the heater/cooler element; pumping a second fluid from the fluid reservoir and through the heat exchanger and the target unit and back to the fluid reservoir, such that the first fluid and the second fluid are maintained as separate fluids; wherein pumping the second fluid facilitates heat transfer in the heat exchanger between the first fluid and the second fluid and heat transfer between the second fluid and the target fluid in the target unit.

Example 19 is the method of Example 18, wherein pumping the second fluid comprises one of pumping the second fluid using a heat transfer fluid pump situated in the heat transfer fluid module and pumping the second fluid using a heat transfer fluid pump situated in the heat exchanger.

Example 20 is the method of Example 18 or 19, wherein pumping the second fluid comprises pumping the second fluid using a reversible pump, the method further comprising reversing the reversible pump to drain the heat exchanger and disinfecting the drained heat exchanger using a thermal disinfection system.

Example 21 is a system including a heater/cooler unit, a fluid reservoir, and a fluid pump. The heater/cooler unit is configured to regulate temperature of a first fluid and pump the first fluid through a heat exchanger in a closed circuit. The fluid pump pumps a second fluid from the fluid reservoir to the heat exchanger and a target unit and back to the fluid reservoir to regulate temperature of the second fluid and the target unit. The fluid pump is a reversible pump including an impeller having flexible blades that bend in a direction opposite to a direction of spin of the impeller in the fluid pump.

Example 22 is the system of Example 21, wherein the reversible pump is configured to drain the second fluid from the heat exchanger and return the second fluid to the fluid reservoir.

While multiple embodiments are disclosed, still other embodiments of the present disclosure will become apparent to those skilled in the art from the following detailed description, which shows and describes illustrative embodiments of the disclosure. Accordingly, the drawings and detailed description are to be regarded as illustrative in nature and not restrictive.

### BRIEF DESCRIPTION OF THE DRAWINGS

Fig. 1 is a diagram illustrating a modular heating/cooling system, according to embodiments of the disclosure.
Fig. 2 is a diagram illustrating another modular heating/cooling system, according to embodiments of the disclosure.
Fig. 3 is a diagram illustrating a heater/cooler unit including a heater/cooler and an integrated heat exchanger, according to embodiments of the disclosure.
Fig. 4 is a diagram illustrating a perspective view of the heat transfer fluid tank, according to embodiments of the disclosure.
Fig. 5 is a diagram illustrating a front view of the heat transfer fluid tank, according to embodiments of the disclosure.
Fig. 6 is a diagram illustrating a back view of the heat transfer fluid tank, according to embodiments of the disclosure.
Fig. 7 is a diagram illustrating a side view of the heat transfer fluid tank, according to embodiments of the disclosure.
Fig. 8 is a diagram illustrating a top view of the heat transfer fluid tank, according to embodiments of the disclosure.
Fig. 9 is a diagram illustrating a cross-section view of the heat transfer fluid tank taken along the line A-A in Fig. 8, according to embodiments of the disclosure.
Fig. 10 is a diagram illustrating a cross-section view of the heat transfer fluid tank taken along the line B-B in Fig. 8, according to embodiments of the disclosure.
Fig. 11 is a diagram illustrating the impeller of the heat transfer fluid pump, according to embodiments of the disclosure.
Fig. 12 is a diagram illustrating the impeller spinning counter-clockwise in a pump casing, according to embodiments of the disclosure.
Fig. 13 is a diagram illustrating the impeller spinning clockwise in the pump casing, according to embodiments of the disclosure.
Fig. 14 is a diagram illustrating the heat transfer fluid tank connected to tubing for connecting the fluid tank to a target unit, according to embodiments of the disclosure.
Fig. 15 is a diagram illustrating the clamp on the tubing in a closed position, according to embodiments of the disclosure.
Fig. 16 is a diagram illustrating the clamp in an open position, according to embodiments of the disclosure.
Fig. 17 is a flow chart diagram illustrating a method of heating/cooling a target fluid, such as blood, in a target unit using the heating/cooling systems of Figs. 1 and 2, according to embodiments of the disclosure.

While the disclosure is amenable to various modifications and alternative forms, specific embodiments have been shown by way of example in the drawings and are described in detail below. The intention, however, is not to limit the disclosure to the particular embodiments described. On the contrary, the disclosure is intended to cover all modifications, equivalents, and alternatives falling within the scope of the disclosure as defined by the appended claims.

### DETAILED DESCRIPTION

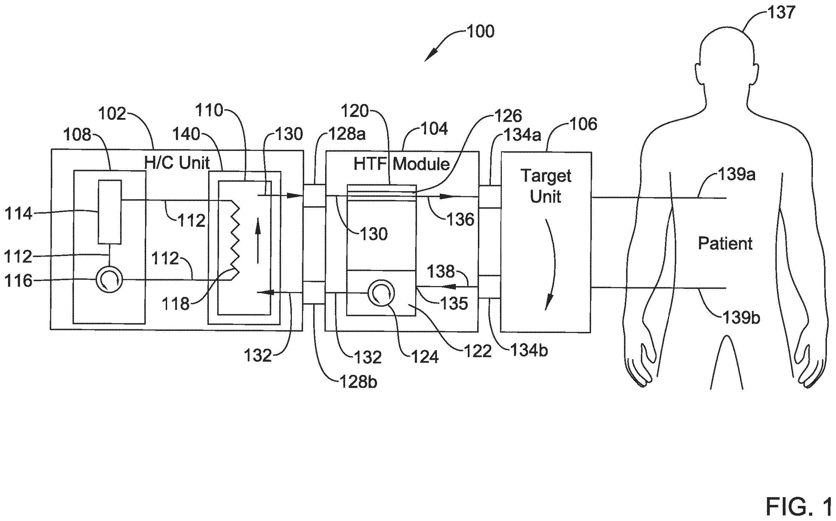

Fig. 1 is a diagram illustrating a modular heating/cooling system 100, according to embodiments of the disclosure. The heating/cooling system 100 includes a heater/cooler unit 102, a disposable heat transfer fluid module 104, and a target unit 106.

The disposable heat transfer fluid module 104 provides the advantage of being disposable, such that the heat transfer fluid module 104 including the heat transfer fluid in the heat transfer fluid module 104 is disposed of to eliminate or reduce the build-up of contaminates and potentially harmful microorganisms in the system. In embodiments, the disposable heat transfer fluid module 104 is a single use module. In embodiments, the heat transfer fluid module 104 includes a transponder, such as transponder 308 discussed below, or a radio frequency identification (RFID) tag that identifies the heat transfer fluid module 104 to the system 100. The transponder or RFID tag can be used to limit the number of uses of the heat transfer fluid module 104 including the heat transfer fluid.

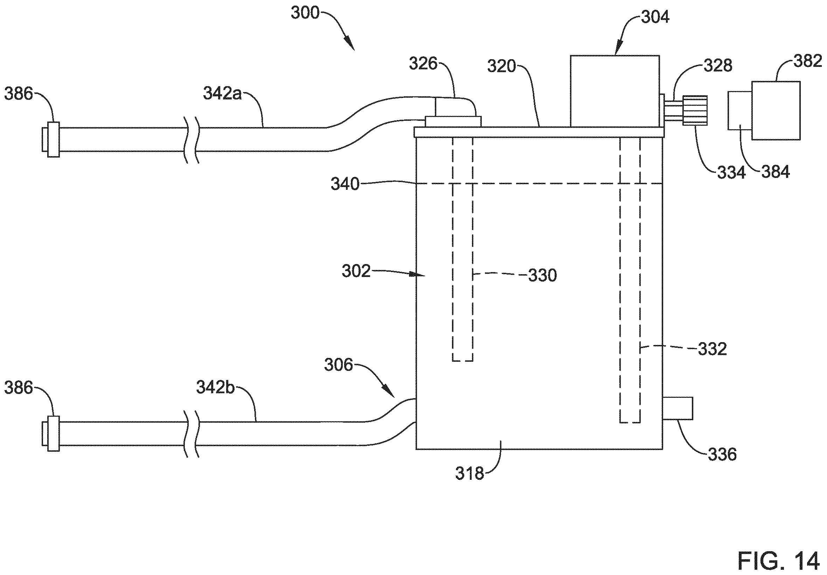

The target unit 106 can include a heat exchanger and, in some embodiments, the target unit 106 includes an oxygenator that includes a heat exchanger. Also, in embodiments, the target unit 106 is located at a target location that may be adjacent the heat transfer fluid module 104, remote from the heat transfer fluid module 104, and/or separate from the heat transfer fluid module 104. In some embodiments, the target unit 106 is at a target location adjacent the heat transfer fluid module 104. In some embodiments, the target unit 106 is at a target location remote from the heat transfer fluid module 104. In embodiments where the target unit 106 is at a target location remote from the heat transfer fluid module 104, the system may use tubing, for example, as illustrated in Fig. 14.

The different parts of the system 100, including the heater/cooler unit 102, the heat transfer fluid module 104, and/or the target unit 106, can be coupled to other similar parts to provide an increase in the heating/cooling capability of the system 100 and/or to increase the number of heating/cooling channels. For example, multiple heater/cooler units 102 may be connected together in series or in parallel and/or multiple heat transfer fluid modules 104 may be connected together in series or in parallel and/or multiple target units 106 may be connected together in series or in parallel to provide an increase in heating/cooling capability and/or an increase in heating/cooling channels. Also, having multiple similar parts provides redundancy in case of failure of any of the parts, and modularity allows the system 100 to be customized to fit different power consumption needs and to provide optimized heating/cooling capabilities, as required for different applications.

In most applications, the system 100 includes one of each of the heater/cooler unit 102, the heat transfer fluid module 104, and/or the target unit 106. In these embodiments, the system 100 consumes 500-600 watts, which makes the system 100 compatible with portable applications, such as ambulance, aircraft, and helicopter applications. Also, low power consumption makes the system 100 compatible with battery operation and with the use of uninterruptible power supplies (UPS's). In addition, low power consumption makes the system 100 compatible with electrical systems in multiple countries, where the system 100 can be plugged into one power outlet without overpowering the single outlet. Thus, the system 100 can be used in Europe where one electrical power outlet may supply up to 3.5 kilowatts, and in the United States where one power outlet may supply 1.8 kilowatts, and in Japan where one power outlet may supply only up to 1.5 kilowatts.

Also, the heating/cooling system 100 has size advantages making it compatible with applications in small areas, such as placing the system 100 or components of the system 100 near a heart lung machine (HLM). In some embodiments, the system 100 takes up an area or volume of only 0.5 x 0.5 x 0.5 meters. This, along with the low power consumption, makes the system 100 available for portable applications, including patient transport in a hospital environment.

The system 100 can be used in different heating/cooling applications in the medical field. These medical field applications include heating/cooling of the blood in an oxygenator, heating/cooling of a drug or drugs in cardioplegia, heating/cooling of clothing or other items such as blankets, hyperthermia and hypothermia procedures, and the heating/cooling of fluids in organ perfusion. In addition, the heating/cooling system 100 can be used in cardiopulmonary bypass (CPB) and extracorporeal membrane oxygenation (ECMO), such as in an intensive care unit (ICU).

The heater/cooler unit 102 includes a heater/cooler 108 and an integrated heat exchanger 110 fluidically coupled to the heater/cooler 108 by heater/cooler tubing 112. The heater/cooler 108 includes a heater/cooler element 114 fluidically coupled to a heater/cooler pump 116 via the heater/cooler tubing 112. The integrated heat exchanger 110 includes a heater/cooler heat exchange element 118 that is fluidically coupled to the heater/cooler element 114 and the heater/cooler pump 116 via the heater/cooler tubing 112. The heater/cooler pump 116 pumps a first fluid through the heater/cooler element 114 and the heater/cooler tubing 112 to and from the heat exchange element 118 in the integrated heat exchanger 110. In some embodiments, the heater/cooler element 114 includes one or more of a heat pump, a resistance heating element, or a thermoelectric heating element. In some embodiments, the heater/cooler pump 116 includes a pump of an HLM and/or a standalone pump. In some embodiments, the first fluid is or includes water, glycol, or combinations of these or other fluids. In some embodiments, the heater/cooler 108 is a permanent part of the heater/cooler unit 102.

In other embodiments, the heater/cooler 108 can be directly connected to the integrated heat exchanger 110, such as by a flange or an extension on one unit that fits into a receptacle on the other unit. In embodiments, these direct connections may reduce the size and footprint of the system. Also, in some embodiments, the heater/cooler 108 can be thermally connected to the integrated heat exchanger 110 such as by heat conducting plates or thermal radiation and not through a first fluid and tubing.

In some embodiments, the heater/cooler tubing 112, the heater/cooler element 114, the heater/cooler pump 116, and the heat exchange element 118 are a closed circuit containing the first fluid that flows through the heater/cooler 108 and the heat exchange element 118 in the integrated heat exchanger 110. In some embodiments, the heater/cooler tubing 112, the heater/cooler element 114, the heater/cooler pump 116, and the heat exchange element 118 are a hermetically sealed closed circuit containing the first fluid. In embodiments where the heater/cooler tubing 112, the heater/cooler element 114, the heater/cooler pump 116, and the heat exchange element 118 are a closed circuit, the system 100 prevents contamination of the OR due to open air tanks holding the first fluid. Also, these embodiments reduce or eliminate the need for disinfecting the heater/cooler tubing 112, the heater/cooler element 114, the heater/cooler pump 116, and the heat exchange element 118 in the integrated heat exchanger 110.

The heat transfer fluid module 104 includes a heat transfer fluid tank 120 that includes a heat transfer fluid reservoir 122, a heat transfer fluid pump 124, and a heat transfer fluid pass through tube 126. In some embodiments, the heat transfer fluid module 104 is a single use disposable module. In some embodiments, the heat transfer fluid pump 124 includes a pump of an HLM and/or a standalone pump. In some embodiments, the heat transfer fluid pump 124 is a reversible pump. In some embodiments, the heat transfer fluid pump 124 is part of the integrated heat exchanger 110 in the heater/cooler unit 102. In some embodiments, the heat transfer fluid pass through tube 126 is not part of the heat transfer fluid tank 120.

The heat transfer fluid module 104 is fluidically coupled to the heater/cooler unit 102 by quick connects/disconnects 128a and 128b, such that the heat transfer fluid pass through tube 126 is fluidically coupled to the integrated heat exchanger 110 through tubing 130 and quick connect/disconnect 128a, and the heat transfer fluid pump 124 is fluidically coupled to the integrated heat exchanger 110 by tubing 132 and quick connect/disconnect 128b.

In other embodiments, the heat transfer fluid module 104 can be directly connected to the heater/cooler unit 102 and the integrated heat exchanger 110. In some embodiments, the heat transfer fluid module 104 can be directly connected to the heater/cooler unit 102 and the integrated heat exchanger 110, such as by the quick connects/disconnects 128a and 128b. In some embodiments, the heat transfer fluid module 104 can be directly connected to the heater/cooler unit 102 and the integrated heat exchanger 110, such as by a flange or an extension on one unit that fits into a receptacle on the other unit. In some embodiments, the quick connects/disconnects 128a and 128b can be snap fit mechanisms having a release button for releasably connecting the heat transfer fluid module 104 to the integrated heat exchanger 110. The heat transfer fluid module 104 is fluidically coupled to the target unit 106 by quick connects/disconnects 134a and 134b, such that the heat transfer fluid pass through tube 126 is fluidically coupled to the target unit 106 through tubing 136 and quick connect/disconnect 134a, and the heat transfer fluid reservoir 122 is fluidically coupled to the target unit 106 through tubing 138 and quick connect/disconnect 134b.

In other embodiments, the heat transfer fluid module 104 can be directly connected to the target unit 106, such as by the quick connects/disconnects 128a and 128b, or such as by a flange or an extension on one unit that fits into a receptacle on the other unit. In some embodiments, the quick connects/disconnects 128a and 128b can be snap fit mechanisms having a release button for releasably connecting the heat transfer fluid module 104 to the target unit 106.

The heat transfer fluid reservoir 122 contains or is filled with a second heat transfer fluid that is pumped through the integrated heat exchanger 110, the heat transfer fluid pass through tube 126, the target unit 106, and back to the heat transfer fluid reservoir 122 by the heat transfer fluid pump 124. In embodiments, the heat transfer fluid pump 124 is a reversible pump, such that the second fluid can be drained from the integrated heat exchanger 110 and/or the target unit 106 and returned to the heat transfer fluid reservoir 122. The heat transfer fluid module 104 can then be disconnected and disposed of, which prevents contamination of the OR due to open air tanks holding the second fluid. In some embodiments, the second fluid is or includes water, glycol, or combinations of these or other fluids .

In embodiments, an integrated thermal disinfection system 140 surrounds the integrated heat exchanger 110 and provides heat to disinfect the integrated heat exchanger 110. In disinfecting, the integrated heat exchanger 110 is emptied of any residual second fluid and hot disinfected at a temperature, such as 95 C, for a specified time to sterilize the integrated heat exchanger 110, which includes the prevention of bacterial growth.

In operation, the heater/cooler pump 116 pumps the first fluid through the heater/cooler element 114 and the heater/cooler tubing 112 to and from the heat exchange element 118 in the integrated heat exchanger 110. The heater/cooler element 114 is controlled to heat/cool the first fluid. Also, the heat transfer fluid pump 124, which in some embodiments is part of the integrated heat exchanger 110 in the heater/cooler unit 102, pumps the second fluid from the heat transfer fluid reservoir 122 through the integrated heat exchanger 110, the heat transfer fluid pass through tube 126, the target unit 106, and back to the heat transfer fluid reservoir 122.

The first fluid and the second fluid remain separated in the system 100, and the temperature of the second fluid is regulated by the temperature of the first fluid. The second fluid is heated/cooled by the heat exchange element 118, where the first fluid flows through the heat exchange element 118. In embodiments, the second fluid flows through the integrated heat exchanger 110 making physical contact with the heat exchange element 118. In embodiments, a different type of integrated heat exchanger 110 can be used to transfer heating/cooling from the first fluid to the second fluid and keep the first and second fluids separated.

In some embodiments, a different type of integrated heat exchanger 110 can be used, such as one or more of the heat exchangers described in publication number WO/2019/068342 having international application number PCT/EP2017/075473, titled "MODULAR HEATER COOLER WITH DISPOSABLE HEAT TRANSFER FLUID CIRCUIT' filed October 6, 2017, which is hereby incorporated by reference in its entirety.

The second fluid flows through the target unit 106 to heat/cool the target unit 106. In some embodiments, the target unit 106 includes a target fluid and the second fluid flows through the target unit 106 to facilitate heat transfer between the second fluid and the target fluid. In embodiments, the target unit 106 includes or is an oxygenator including a heat exchanger for heating/cooling blood, such that the second fluid flows through the heat exchanger of the oxygenator to heat/cool the blood, which is the target fluid.

In some embodiments, the target unit 106 includes or is an oxygenator fluidically connected to a patient 137 by blood fluid lines 139a and 139b. The oxygenator includes a heat exchanger that receives blood from the patient 137 as the target fluid and that receives the second fluid. In embodiments, the blood flows from the patient 137 and through the blood fluid line 139a to the oxygenator, including to the heat exchanger in the oxygenator, and the blood flows through the blood fluid line 139b from the oxygenator and the heat exchanger back to the patient 137. The temperature of the blood is regulated by heat transfer between the second fluid and the blood in the heat exchanger of the oxygenator.

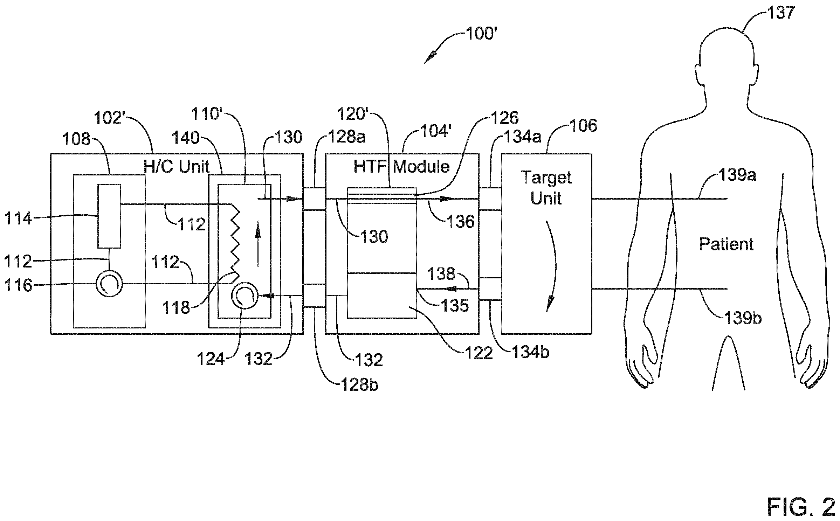

Fig. 2 is a diagram illustrating another modular heating/cooling system 100', according to embodiments of the disclosure. The heating/cooling system 100' is the same as the heating/cooling system 100 except the heat transfer fluid pump 124 has been moved from the heat transfer fluid tank 120 in the disposable heat transfer fluid module 104 to the integrated heat exchanger 110 in the heater/cooler unit 102. Thus, the heating/cooling system 100' includes a heat transfer fluid tank 120' in a disposable heat transfer fluid module 104' that does not include the heat transfer fluid pump 124 and an integrated heat exchanger 110' in a heater/cooler unit 102' that does include the heat transfer fluid pump 124. The other numbered components in Fig. 2 are the same in form and function as like numbered components in Fig. 1, and the heating/cooling system 100' functions and operates the same as the heating/cooling system 100.

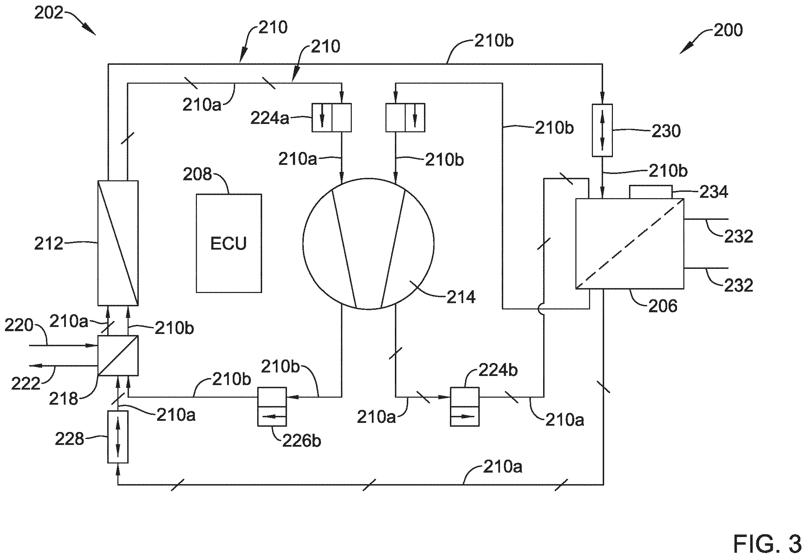

Fig. 3 is a diagram illustrating a heater/cooler unit 200 including a heater/cooler 202 and an integrated heat exchanger 206, according to embodiments of the disclosure. In embodiments, the heater/cooler unit 200 is similar to the heater/cooler unit 102 (shown in Fig. 1) or the heater/cooler unit 102' (shown in Fig. 2). In some embodiments, the heater/cooler 202 is similar to the heater/cooler 108 and, in some embodiments, the heat exchanger 206 is similar to the integrated heat exchanger 110 (shown in Fig. 1) or the integrated heat exchanger 110' (shown in Fig. 2).

The heater/cooler 202 includes an electronic control unit 208 and a primary circuit 210 for heating and cooling the first fluid in the primary circuit 210. The electronic control unit 208 can be one or more of a controller, a processor, a micro-controller, a micro-processor, and a computer. Also, the electronic control unit 208 can include memory, a user interface having input and output portions, such as a touch screen display, and executable code stored in memory that the electronic control unit 208 executes to control the components of the heater/cooler 202. The primary circuit 210 includes heating circuit tubing 21Oa, indicated by slashes on the tubing 21Oa, in a heating circuit path for heating the first fluid, and cooling circuit tubing 21Ob, indicated with non-slashed tubing 21Ob, in a cooling circuit path for cooling the first fluid. In some embodiments, the first fluid includes water. In some embodiments, the primary circuit 210 is a permanent part of the heater/cooler 202.

In some embodiments, the primary circuit 210, including the heating circuit path and the cooling circuit path, is a closed circuit containing the first fluid. In some embodiments, the primary circuit 210, including the heating circuit path and the cooling circuit path, is a hermetically sealed closed circuit containing the first fluid. In embodiments where the primary circuit 210 is a closed circuit, the heater/cooler unit 200 prevents contamination of the OR due to open air tanks holding the first fluid. Also, these embodiments eliminate the need for disinfecting the primary circuit 210.

The primary circuit 210 includes a heater/cooler element 212, a heater/cooler primary circuit pump 214, part of the heat exchanger 206 and, optionally, an auxiliary heat exchanger 218. In some embodiments, the heater/cooler element 212 includes a heat pump. In some embodiments, the primary circuit pump 214 includes a pump of an HLM and/or a standalone pump. In some embodiments, the auxiliary heat exchanger 218 receives a heat exchanger fluid at 220 and transmits the heat exchanger fluid at 222. The heat exchanger fluid is pumped through the auxiliary heat exchanger 218 to facilitate heat transfer between the heat exchanger fluid and the first fluid.

The primary circuit path 210 also includes heating circuit valves 224a and 224b and cooling circuit valves 226a and 226b. In addition, the primary circuit path 210 includes a heating circuit expansion valve 228 and a cooling circuit expansion valve 230. The electronic control unit 208 is electrically coupled to the heater/cooler element 212, the primary circuit pump 214, the heat exchanger 206, the auxiliary heat exchanger 218, the heating circuit valves 224a and 224b, the cooling circuit valves 226a and 226b, the heating circuit expansion valve 228, and the cooling circuit expansion valve 230 to control operation of the heater/cooler 202.

In the heating circuit path, the heating circuit tubing 21Oa fluidically couples the following components together: the heater/cooler element 212 is fluidically coupled to the heating circuit valve 224a that is fluidically coupled to the primary circuit pump 214 that is fluidically coupled to the heating circuit valve 224b that is fluidically coupled to the heat exchanger 206 that is fluidically coupled to the heating circuit expansion valve 228 that is fluidically coupled to the auxiliary heat exchanger 218 that is fluidically coupled to the heater/cooler element 212.

In the cooling circuit path, the cooling circuit tubing 21Ob fluidically couples the following components together: the heater/cooler element 212 is fluidically coupled to the cooling circuit expansion valve 230 that is fluidically coupled to the heat exchanger 206 that is fluidically coupled to the cooling circuit valve 226a that is fluidically coupled to the primary circuit pump 214 that is fluidically coupled to the cooling circuit valve 226b that is fluidically coupled to the auxiliary heat exchanger 218 that is fluidically coupled to the heater/cooler element 212.

In heating the first fluid, the primary circuit pump 214 pumps the first fluid through the heating circuit path including the heating circuit valve 224b to the heat exchanger 206 to the heating circuit expansion valve 228 to the auxiliary heat exchanger 218 to the heater/cooler element 212 to the heating circuit valve 224a and back to the primary circuit pump 214. The primary circuit pump 214 and the heater/cooler element 212 are controlled by the electronic control unit 208 to heat the first fluid. Also, optionally, the auxiliary heat exchanger 218 is controlled, such as by the electronic control unit 208, to heat the first fluid.

In cooling the first fluid, the primary circuit pump 214 pumps the first fluid through the cooling circuit path including the cooling circuit valve 226b to the auxiliary heat exchanger 218 to the heater/cooler element 212 to the cooling circuit expansion valve 230 to the heat exchanger 206 to the cooling circuit valve 226a and back to the primary circuit pump 214. The primary circuit pump 214 and the heater/cooler element 212 are controlled by the electronic control unit 208 to cool the first fluid. Also, optionally, the auxiliary heat exchanger 218 is controlled, such as by the electronic control unit 208, to cool the first fluid.

The heat exchanger 206 includes a heat exchange element, such as heat exchange element 118, that is part of the primary circuit 210 through which the first fluid flows. The heat exchanger 206 also includes part of a secondary circuit 232 through which a second fluid flows to facilitate heat transfer between the first fluid and the second fluid in the heat exchanger 206. The temperature of the second fluid is regulated by the temperature of the first fluid. In some embodiments, the heat exchanger 206 includes a thermoelectric heater/cooler 234 thermally coupled to the heat exchanger 206 to heat and/or cool at least one of the first fluid and the second fluid. In some embodiments, the thermoelectric heater/cooler 234 is controlled by the electronic control unit 208. In some embodiments, a target unit, such as target unit 106, includes a target fluid and the second fluid flows through the target unit to facilitate heat transfer between the second fluid and the target fluid.

In some embodiments, the heat exchanger 206 includes one or more auxiliary electric heaters configured to heat the first fluid in the heat exchanger 206. In some embodiments, the heat exchanger 206 includes one or more auxiliary electric heaters configured to heat the second fluid in the heat exchanger 206. In some embodiments, the heat exchanger 206 includes one or more auxiliary electric heaters configured to be used during thermal disinfection to dry and thermally disinfect the heat exchanger 206. In some embodiments, one or more auxiliary electric heaters in the heat exchanger 206 are controlled by the electronic control unit 208.

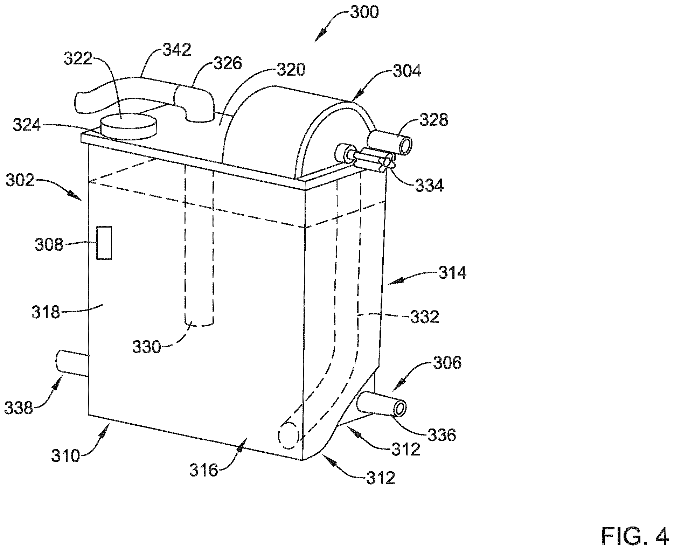

Figures 4-16 are diagrams illustrating an exemplary heat transfer fluid tank 300, according to embodiments of the disclosure. The fluid tank 300 can be used in the heat transfer fluid module 104 (shown in Fig. 1). In embodiments, the fluid tank 300 is similar to the heat transfer fluid tank 120 (shown in Fig. 1).

Fig. 4 is a diagram illustrating a perspective view of the heat transfer fluid tank 300, according to embodiments of the disclosure. The fluid tank 300 includes a heat transfer fluid reservoir 302, a reversible heat transfer fluid pump 304, and a heat transfer fluid pass through tube 306. The fluid tank 300 also includes connections for connecting the fluid tank 300 to other modules, such as the heater/cooler unit 102 and the target unit 106. In some embodiments, the heat transfer fluid tank 300 includes a transponder 308 for wireless communications to a base system for identifying the fluid tank 300 to the base system and preventing off label use, e.g., reuse of the fluid tank 300 instead of replacement. In some embodiments, the fluid reservoir 302 is similar to the heat transfer fluid reservoir 122. In some embodiments, the fluid pump 304 is similar to the heat transfer fluid pump 124. In some embodiments, the fluid pass through tube 306 is similar to the heat transfer fluid pass through tube 126.

The heat transfer fluid tank 300 as shown in the figures is shaped like a rectangular prism or rectangular box having a flat bottom 310, such that the fluid tank 300 is stable standing upright. In other embodiments, the fluid tank 300 can be another suitable shape such as cylindrical, cuboid, or a triangular prism.

The heat transfer fluid reservoir 302 as shown in the figures is shaped like a rectangular prism or rectangular box with a reservoir bottom 312 that is angled down from the back 314 of the fluid tank 300 to the front 316 of the fluid tank 300. This angled reservoir bottom 312 causes the fluid in the fluid reservoir 302 to flow to the deeper front portion of the fluid reservoir 302. Also, the angled reservoir bottom 312 makes room outside the fluid reservoir 302 for the heat transfer fluid pass through tube 306 to be attached to the fluid tank 300.

The heat transfer fluid reservoir 302 includes a body 318 and a top 320 that is secured to and sealed to the body 318. The fluid tank 300 further includes a fill cap 322 that is removably secured to the top 320, such as by screwing or turning the fill cap 322 into a fill cap location 324 in the top 320. The fluid reservoir 302 is filled with heat transfer fluid through the fill cap location 324 in the top 320 and the fill cap 322 is then secured to the top 320.

The top 320 further includes a target unit connection 326 for connecting the fluid tank 300 to a target unit, such as target unit 106, and a heater/cooler connection 328 for connecting the fluid tank 300 to a heater /cooler module, such as heater/cooler unit 102. The target unit connection 326 is fluidically coupled to or formed integral with a fluid return tube 330 that is internal to the fluid reservoir 302 and extends toward the bottom of the fluid reservoir 302.

The heat transfer fluid pump 304 is secured to the top 320 and fluidically coupled to the heater/cooler connection 328. Also, the fluid pump 304 is fluidically coupled to an internal fluid pick-up tube 332 that extends to the deeper front bottom portion of the heat transfer fluid reservoir 302. The fluid pump 304 includes a drive shaft 334 configured to be coupled to a drive motor, which may be a drive motor that is part of a heater/cooler unit, which turns the drive shaft 334 in forward and reverse directions as needed.

In embodiments, one end of the heat transfer fluid pass through tube 306 includes a heater/cooler connection 336 that is fluidically coupled to an integrated heat exchanger, such as the integrated heat exchanger 110, and the other end, indicated at 338, of the heat transfer fluid pass through tube 306 is fluidically coupled to a target unit, such as target unit 106. The heat transfer fluid pump 304 is fluidically coupled to the integrated heat exchanger through the other heater/cooler connection 328 and the target unit connection 326 is fluidically coupled to the target unit.

In operation, the heat transfer fluid tank 300 is fluidically coupled to a heater/cooler unit, such as heater/cooler unit 102, and fluidically coupled to a target unit, such as target unit 106. The heat transfer fluid reservoir 302 is filled with a heat transfer fluid through the fill cap location 324 in the top 320 and the fill cap 322 is secured to the top 320. The heat transfer fluid pump 304 is driven in a forward direction to draw heat transfer fluid through the internal fluid pick-up tube 332 and pump the heat transfer fluid from the fluid reservoir 302 out of the heater/cooler connection 328 and through the integrated heat exchanger and the heat transfer fluid pass through tube 306 to a target unit. The heat transfer fluid returns from the target unit to the fluid reservoir 302 through the target unit connection 326 and the fluid return tube 330.

In embodiments, to drain or empty the heat transfer fluid from the connected modules, such as the heater/cooler unit 102 and the target unit 106, the target unit is disconnected from the target unit connection 326 and the heat transfer fluid pump 304 is driven in a reverse direction to pull the heat transfer fluid from the integrated heat exchanger and the target unit and back into the fluid reservoir 302 through the internal fluid pick-up tube 332. The heat exchanger, such as the integrated heat exchanger 110, can then be disinfected.

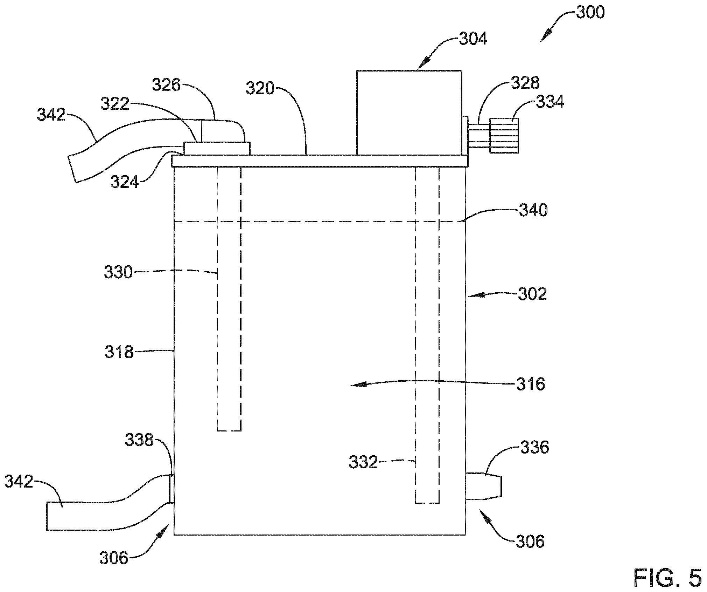

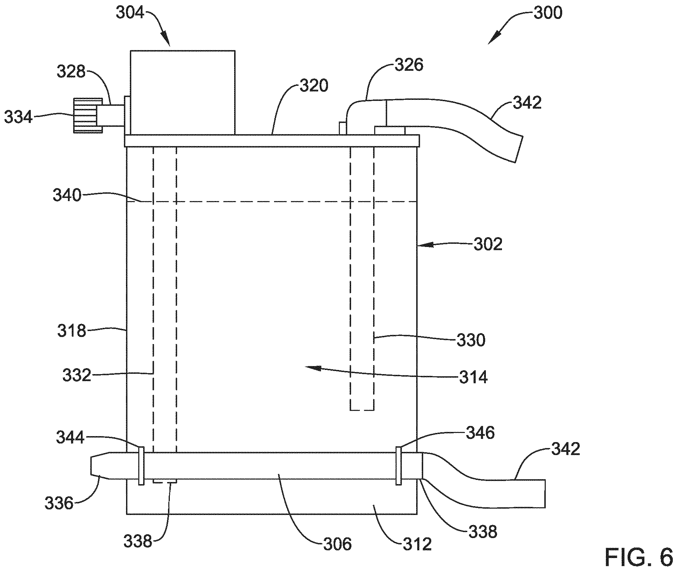

Fig. 5 is a diagram illustrating a front view of the heat transfer fluid tank 300, according to embodiments of the disclosure, and Fig. 6 is a diagram illustrating a back view of the heat transfer fluid tank 300, according to embodiments of the disclosure. The front view illustrated in Fig. 5 shows the front 316 of the fluid tank 300 and the back view illustrated in Fig. 6 shows the back 314 of the fluid tank 300.

In reference to Figs. 5 and 6, the heat transfer fluid tank 300 includes the heat transfer fluid reservoir 302, the reversible heat transfer fluid pump 304, and the heat transfer fluid pass through tube 306. The fluid reservoir 302 includes the body 318 and the top 320, and the fluid tank 300 includes the fill cap 322 removably secured to the top 320 at the fill cap location 324. Also, the fluid reservoir 302 is filled with heat transfer fluid up to the dashed line 340.

The top 320 includes the target unit connection 326 for connecting the fluid tank 300 to a target unit and the heater/cooler connection 328 for connecting the fluid tank 300 to a heater/cooler unit. The target unit connection 326 is fluidically coupled to the fluid return tube 330 and the heater/cooler connection 328 is fluidically coupled to the heat transfer fluid pump 304 that is fluidically coupled to the internal fluid pick-up tube 332. The fluid pump 304 includes the drive shaft 334 configured to be coupled to a drive motor that turns the drive shaft 334 in forward and reverse directions.

In further reference to Figs. 5 and 6, the heat transfer fluid tank 300 has a front view profile that is rectangular and a back view profile that is rectangular. The back view profile includes the angled reservoir bottom 312 that makes room for the heat transfer fluid pass through tube 306 outside the fluid reservoir 302. The fluid pass through tube 306 includes the target unit connection 338 and the heater/cooler connection 336. The target unit connection 326 of the top 320 and the target unit connection 338 of the pass through tube 306 are each connected to tubing 342 that is further connected to the target unit. In embodiments, the fluid pass through tube 306 is attached to the fluid tank 300 at one end by an attachment mechanism 344 and at the other end by a second attachment mechanism 346. In other embodiments, the fluid pass through tube 306 can be attached to the fluid tank 300 in other ways, such as by inserting the tube 306 through a cavity and clamping the tube 306 in place.

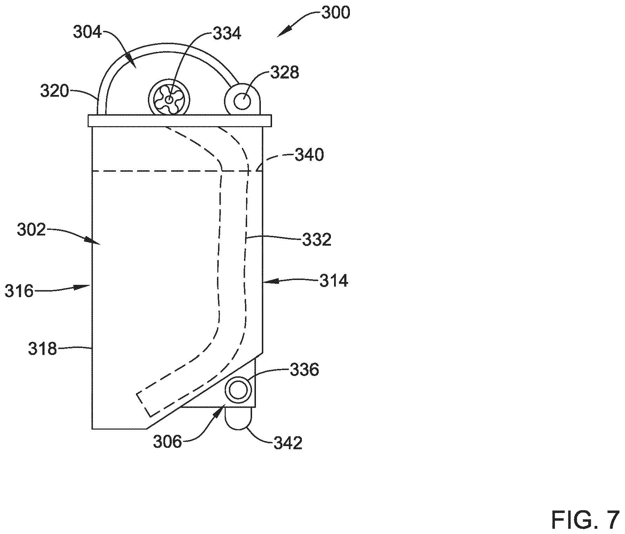

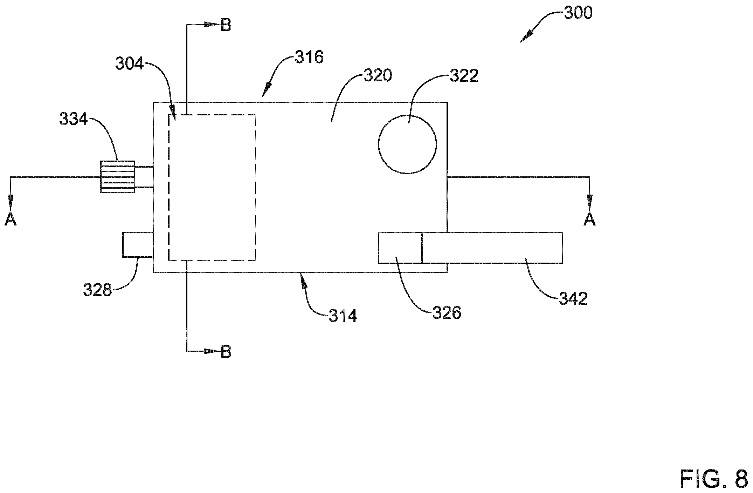

Fig. 7 is a diagram illustrating a side view of the heat transfer fluid tank 300, according to embodiments of the disclosure, and Fig. 8 is a diagram illustrating a top view of the heat transfer fluid tank 300, according to embodiments of the disclosure. The heat transfer fluid tank 300 has a side view profile that is rectangular and a top view profile that is rectangular. Each of the views of Figs. 7 and 8 show the front 316 and the back 314 of the fluid tank 300. The side view illustrated in Fig. 7 shows the side with the heat transfer fluid pump 304 of the fluid tank 300, and the top view illustrated in Fig. 8 shows the top 320 of the fluid tank 300.

In reference to Figs. 7 and 8, the heat transfer fluid tank 300 includes the heat transfer fluid reservoir 302, the reversible heat transfer fluid pump 304, and the heat transfer fluid pass through tube 306. The fluid reservoir 302 includes the body 318 and the top 320, and the fluid tank 300 includes the fill cap 322 removably secured to the top 320. Also, the fluid reservoir 302 is filled with heat transfer fluid up to the dashed line 340.

The top 320 includes the target unit connection 326 for connecting the fluid tank 300 to a target unit and the heater/cooler connection 328 for connecting the fluid tank 300 to a heater/cooler unit. The heater/cooler connection 328 is fluidically coupled to the heat transfer fluid pump 304 that is fluidically coupled to the internal fluid pick-up tube 332. The fluid pump 304 includes the drive shaft 334 configured to be coupled to a drive motor that turns the drive shaft 334 in forward and reverse directions.

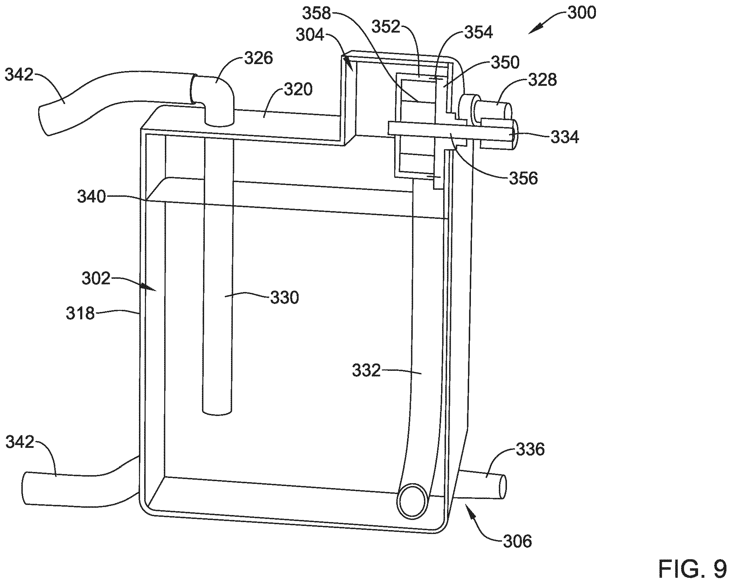

Fig. 9 is a diagram illustrating a cross-section view of the heat transfer fluid tank 300 taken along the line A-A in Fig. 8, according to embodiments of the disclosure. The heat transfer fluid tank 300 includes the heat transfer fluid reservoir 302, the reversible heat transfer fluid pump 304, and the heat transfer fluid pass through tube 306. The fluid reservoir 302 includes the body 318 and the top 320 and is filled with heat transfer fluid up to the line at 340.

The top 320 includes the target unit connection 326 for connecting the fluid tank 300 to a target unit and the heater/cooler connection 328 for connecting the fluid tank 300 to a heater/cooler unit. The target unit connection 326 is fluidically coupled to tubing 342 and to the fluid return tube 330 and the heater/cooler connection 328 is fluidically coupled to the heat transfer fluid pump 304 that is fluidically coupled to the internal fluid pick-up tube 332. Also, the fluid pass through tube 306 includes the heater/cooler connection 336 and tubing 342 connected to the target unit connection 338 (not shown in Fig. 9).

The fluid pump 304 includes the drive shaft 334 that is configured to be coupled to a drive motor that turns the drive shaft 334 in forward and reverse directions. As seen in the cross section, the fluid pump 304 includes a pump front 350 secured to a pump casing 352 by devices 354, such as screws. The drive shaft 334 includes an internal drive shaft portion 356 that is attached to an impeller 358. The drive shaft portion 356 extends through the pump front 350 and to the end of the drive shaft 334, which is configured to be coupled to a drive motor. The drive shaft 334 is turned by the drive motor, which turns the impeller 358 to pump the heat transfer fluid in and out of the fluid reservoir 302.

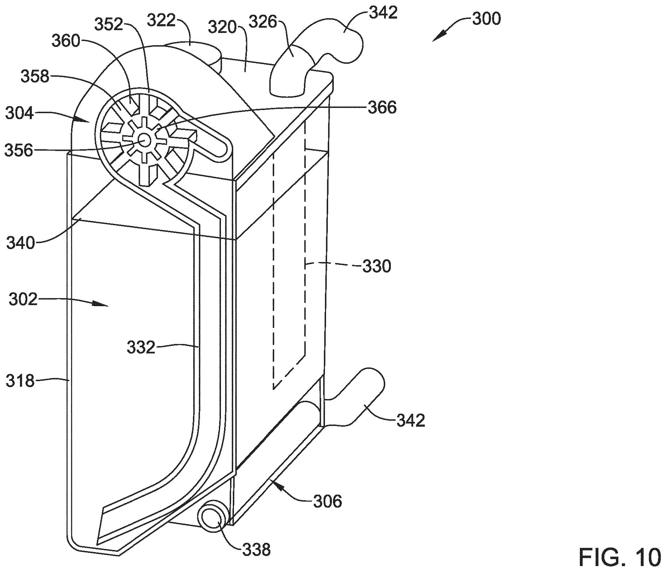

Fig. 10 is a diagram illustrating a cross-section view of the heat transfer fluid tank 300 taken along the line B-B in Fig. 8, according to embodiments of the disclosure. Briefly, the heat transfer fluid tank 300 includes the heat transfer fluid reservoir 302, the reversible heat transfer fluid pump 304, and the heat transfer fluid pass through tube 306. The fluid reservoir 302 includes the body 318 and the top 320 and is filled with heat transfer fluid up to the line at 340.

The top 320 includes the target unit connection 326 for connecting the fluid tank 300 to a target unit and the fill cap 322. The target unit connection 326 is fluidically coupled to tubing 342 and to the fluid return tube 330 and the heater/cooler connection 328 (not shown in Fig. 10) is fluidically coupled to the heat transfer fluid pump 304 that is fluidically coupled to the internal fluid pick-up tube 332. Also, the fluid pass through tube 306 includes the target unit connection 338 connected to tubing 342.

The fluid pump 304 includes the pump casing 352 and the impeller 358 secured to the internal drive shaft portion 356. The impeller 358 includes flexible impeller blades 360 that are turned inside the pump casing 352 to pump the heat transfer fluid in and out of the fluid reservoir 302.

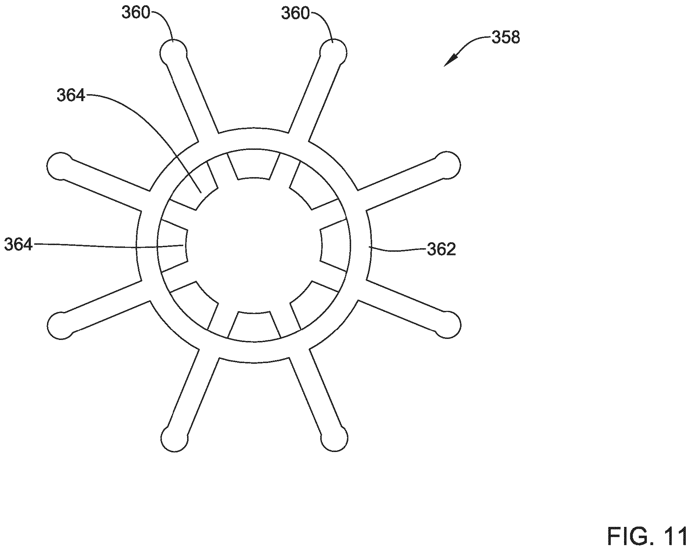

Fig. 11 is a diagram illustrating the impeller 358 of the heat transfer fluid pump 304, according to embodiments of the disclosure. The impeller 358 includes the flexible impeller blades 360, an impeller body 362, and impeller teeth 364. The impeller 358 includes a flexible material, such as rubber or plastic. In embodiments, the impeller 358 is made from a flexible material, such as rubber or plastic. In some embodiments, the impeller 358 including the impeller blades 360, the impeller body 362, and the impeller teeth 364 are formed as one integrated unit in a molding process.

The impeller body 362 has a circular profile with the impeller teeth 364 connected to or integral with the impeller body 362 and spaced apart around the interior circumference of the impeller body 362. The impeller 358 is inserted onto the internal drive shaft portion 356 of the drive shaft 334, such that the impeller teeth 364 mate with corresponding drive shaft teeth 366 (as shown in Fig. 10) on the internal drive shaft portion 356. Thus, the impeller 358 spins with the drive shaft 334 and the impeller teeth 364 and drive shaft teeth 366 prevent the impeller 358 from slipping on the drive shaft 334.

The flexible impeller blades 360 are connected to or integral with the impeller body 362 and spaced apart around the exterior circumference of the impeller body 362. The impeller blades 360 turn inside the pump casing, such as pump casing 352, to pump the heat transfer fluid in and out of the fluid reservoir 302.

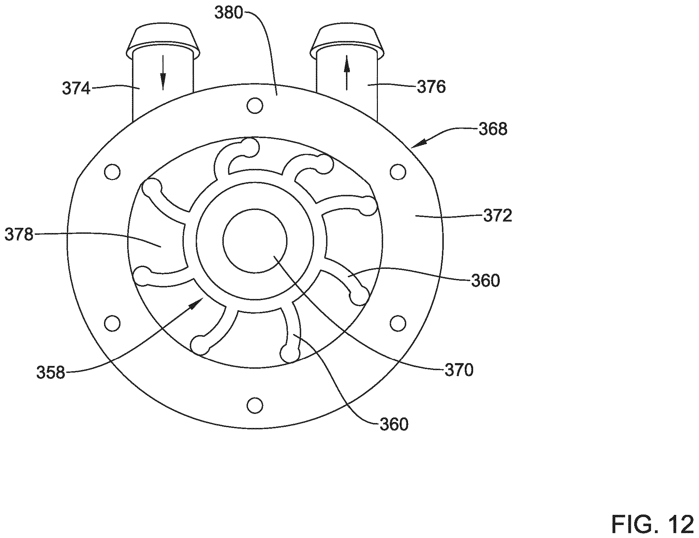

Fig. 12 is a diagram illustrating the impeller 358 spinning counter-clockwise in a pump casing 368, according to embodiments of the disclosure. The impeller 358 is securely fit or mounted on a drive shaft 370 that is spinning in the counter-clockwise direction.

The pump casing 368 includes a pump body 372, a first port 374, and a second port 376. The pump body 372 has an interior cavity 378, such that each of the first port 374 and the second port 376 has a hole through it that extends from the interior cavity 378 and through the pump body 372 and each of the ports 374 and 376 to outside the pump casing 368. The interior cavity 378 has a generally circular interior circumference with a flattened portion 380 near the first port 374 and the second port 376.

In operation, as the impeller 358 spins in the counter-clockwise direction, the impeller blades 360 are bent clockwise at the flattened portion 380 to expel or push fluid out of the second port 376. Also, as the impeller blades 360 spin in the counter-clockwise direction, the impeller blades 360 leave the flattened portion 380 and extend out to the circular interior circumference of the pump casing 368 to create suction and pull fluid into the first port 374.

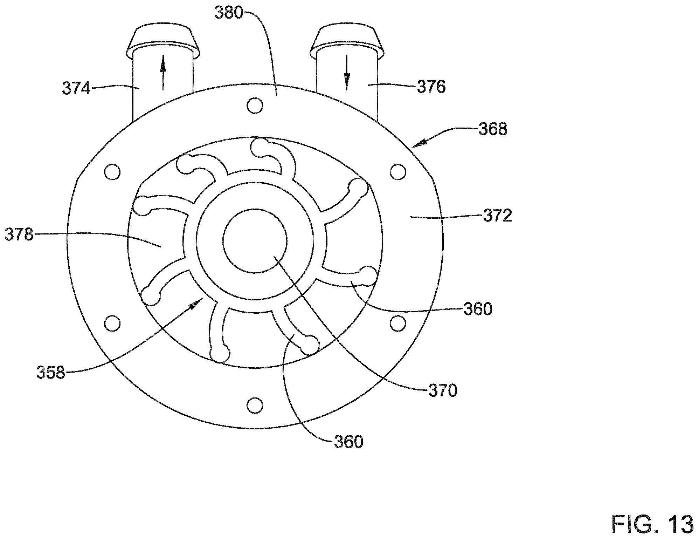

Fig. 13 is a diagram illustrating the impeller 358 spinning clockwise in the pump casing 368, according to embodiments of the disclosure. The impeller 358 is mounted on the drive shaft 370 that is spinning in a clockwise direction.

In operation, as the impeller 358 spins in the clockwise direction, the impeller blades 360 are bent counter-clockwise at the flattened portion 380 to expel or push fluid out of the first port 374. Also, as the impeller blades 360 spin in the clockwise direction, the impeller blades 360 leave the flattened portion 380 and extend out to the circular interior circumference of the pump casing 368 to create suction and pull fluid into the second port 376.

Fig. 14 is a diagram illustrating the heat transfer fluid tank 300 connected to tubing 342 for connecting the fluid tank 300 to a target unit, according to embodiments of the disclosure. In embodiments, the target unit is target unit 106 (shown in Fig. 1).

The heat transfer fluid tank 300 includes the heat transfer fluid reservoir 302, the reversible heat transfer fluid pump 304, and the heat transfer fluid pass through tube 306. The fluid reservoir 302 includes the body 318 and the top 320, and the fluid reservoir 302 is filled with heat transfer fluid up to the dashed line 340.

The top 320 includes the target unit connection 326 for connecting the fluid tank 300 to a target unit and the heater/cooler connection 328 for connecting the fluid tank 300 to a heater/cooler unit. The target unit connection 326 is fluidically coupled to the fluid return tube 330 and the heater/cooler connection 328 is fluidically coupled to the heat transfer fluid pump 304 that is fluidically coupled to the internal fluid pick-up tube 332.

The fluid pump 304 includes the drive shaft 334 configured to be coupled to a drive motor 382 that turns the drive shaft 334 in forward and reverse directions. In embodiments, the drive shaft 334 includes teeth that engage corresponding teeth in a motor drive shaft connector 384. In embodiments, the drive motor 382 is part of a heater/cooler unit, such as heater/cooler unit 102.

The target unit connection 326 is connected to a first length 342a of the tubing 342 (e.g., a coil of tubing), such as by an interference fit. This secures the first length 342a of the tubing 342 to the target unit connection 326 in a watertight or fluid-tight fit. Also, the target unit connection 338 (not shown in Fig. 14) of the pass through tube 306 is connected to a second length 342b of the tubing 342 (e.g., a coil of tubing), such as by interference fit, to secure the second length 342b of the tubing 432 to the target unit connection 338 in a watertight or fluid-tight fit. In embodiments each of the first length of tubing 342a and the second length of tubing 342b is up to 8 meters in length.

A clamp 386 is slid onto or attached to each of the first and second coils 342a and 342b of the tubing 342 to clamp the tubing 342 to the target unit in a watertight or fluid-tight manner.

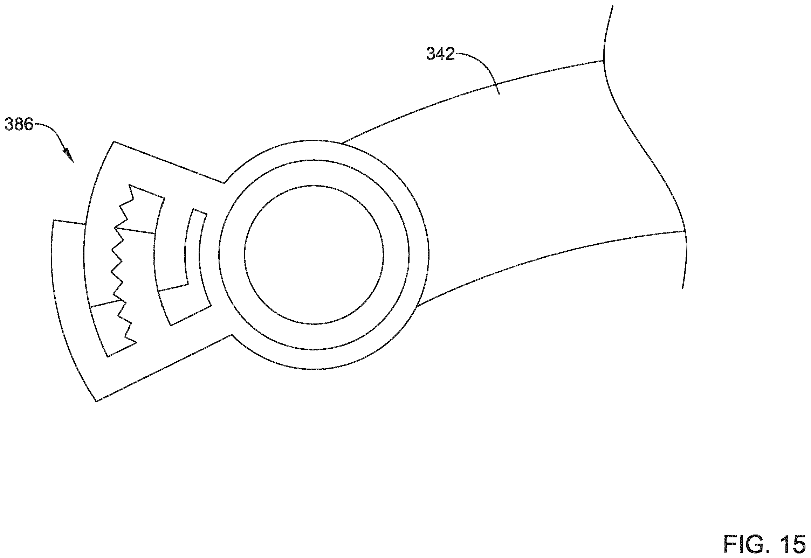

Fig. 15 is a diagram illustrating the clamp 386 on the tubing 342 in a closed position, according to embodiments of the disclosure. The clamp 386 has been squeezed together to lock the clamp 386 in place at the end of the tubing 342.

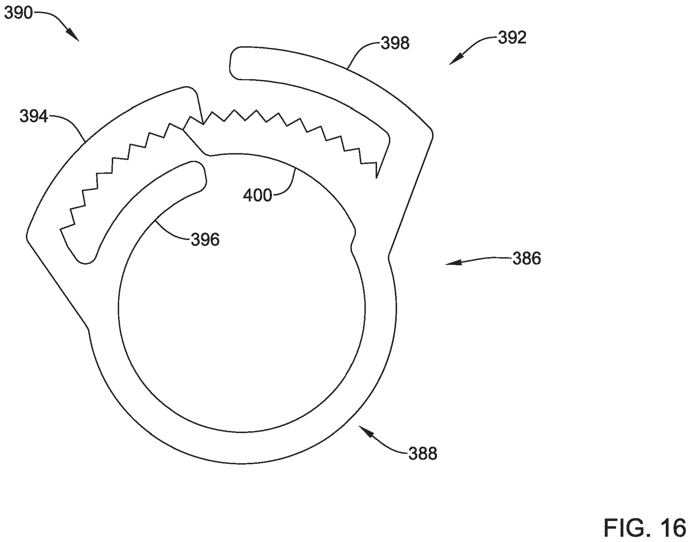

Fig. 16 is a diagram illustrating the clamp 386 in an open position, according to embodiments of the disclosure. The clamp 386 includes a circular portion 388 that has a first clamp portion 390 at one end and a second clamp portion 392 at the other end of the circular portion 388. Each of the first and second clamp portions 390 and 392 is a c-shaped claw configured to engage the other clamp portion. The first clamp portion 390 includes a serrated or teethed top portion 394 and a smooth bottom portion 396, and the second clamp portion 392 includes a smooth top portion 398 and a serrated or teethed bottom portion 400.

To close the clamp 386, the serrated top portion 394 of the first clamp portion 390 is slid between the top and bottom portions 398 and 400 of the second clamp portion 392, and the serrated bottom portion 400 of the second clamp portion 392 is slid between the top and bottom portions 394 and 396 of the first clamp portion 390. The serrated or teethed top portion 394 of the first clamp portion 390 engages the serrated or teethed bottom portion 400 of the second clamp portion 392 to secure the clamp 386 in the closed position.

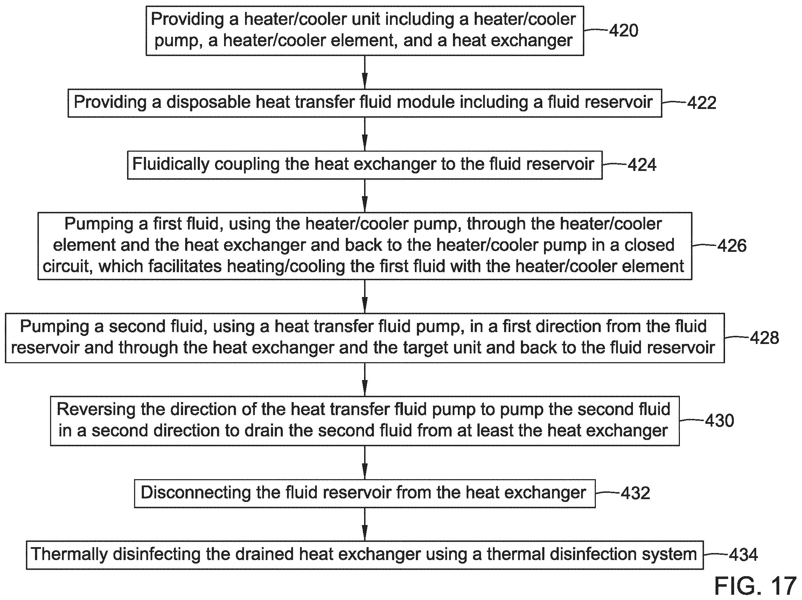

Fig. 17 is a flow chart diagram illustrating an exemplary method of heating/cooling a target fluid, such as blood, in a target unit 106 using the heating/cooling systems 100 and 100' of Figs. 1 and 2, according to embodiments of the disclosure.

At 420, the method includes providing a heater/cooler unit 102/102' including a heater/cooler pump 116, a heater/cooler element 114, and a heat exchanger 110/110'. At 422, the method includes providing a disposable heat transfer fluid module 104/104' including a fluid reservoir 122.

At 424, the method includes fluidically coupling the heat exchanger 110/110' of the heater/cooler unit 102/102' to the fluid reservoir 122 of the heat transfer fluid module 104/104'. In embodiments, the heat exchanger 110/110' of the heater/cooler unit 102/102' is connected to the fluid reservoir 122 of the heat transfer fluid module 104/104' by tubing 132. In embodiments, the heat exchanger 110/110' of the heater/cooler unit 102/102' is directly connected to the fluid reservoir 122 of the heat transfer fluid module 104/104'. In some embodiments, the heat exchanger 110/110' of the heater/cooler unit 102/102' is directly connected to the fluid reservoir 122 of the heat transfer fluid module 104/104' by quick connects/disconnects, such as by one or more of quick connects/disconnects 128a and 128b. In some embodiments, the heat exchanger 110/110' of the heater/cooler unit 102/102' is directly connected to the fluid reservoir 122 of the heat transfer fluid module 104/104', such as by a flange or an extension on one unit that fits into a receptacle on the other unit. In some embodiments, one or more of the quick connects/disconnects is a snap fit mechanism having a release button for releasably connecting the heater/cooler unit 102 and the heat transfer fluid module 104.

At 426, the method includes pumping a first fluid, using the heater/cooler pump 116 in the heater/cooler unit 102, through the heater/cooler element 114 to the heat exchange element 118 in the integrated heat exchanger 110 and back to the heater/cooler pump 116. The heater/cooler element 114 is controlled to heat/cool the first fluid.

At 428, the method includes pumping a second fluid in the disposable heat transfer fluid module 104 from the heat transfer fluid reservoir 122 and through the integrated heat exchanger 110, which facilitates heat transfer between the first fluid and the second fluid. The second fluid is further pumped from the integrated heat exchanger 110 through the pass through tube 126 to the target unit 106 and back to the heat transfer fluid reservoir 122.

The first fluid and the second fluid remain separated in the integrated heat exchanger 110 and the temperature of the second fluid is regulated by the temperature of the first fluid. In some embodiments, the second fluid flows through the integrated heat exchanger 110 making physical contact with the heat exchange element 118. In other embodiments, a different type of integrated heat exchanger 110 is used to transfer heating/cooling from the first liquid to the second fluid and keep the first and second fluids separated.

The second fluid flows through the target unit 106 to heat/cool the target fluid, such as blood, in the target unit 106. The second fluid flows through the target unit 106 to facilitate heat transfer between the second fluid and the target fluid. In embodiments, the target unit 106 includes or is an oxygenator including a heat exchanger for heating/cooling blood, such that the second fluid flows through the heat exchanger of the oxygenator to heat/cool the blood and the second fluid is maintained separate from the target fluid.

At 430, the method includes reversing the heat transfer fluid pump 124 to drain or empty the second fluid from at least the heat exchanger 110/110'. Reversing the heat transfer fluid pump 124 pulls the second fluid from the heat exchanger 110/110' and, if connected, from the target unit 106 to drain or empty the second fluid from the heat exchanger 110/110' and, if connected, the target unit 106. The second fluid is returned to the fluid reservoir 122. In embodiments, the input 135 to the fluid reservoir 122 from the target unit 106 is closed off, such as by a valve (not shown), prior to reversing the heat transfer fluid pump 124. In embodiments, the target unit 106 is disconnected at quick connect/disconnect 134b and this closes off the disconnect 134b, prior to reversing the heat transfer fluid pump 124. In some embodiments, step 430 is not performed.

At 432, the method includes disconnecting the fluid reservoir 122 from the heat exchanger 110/110' and the heater/cooler unit 102/102'. At 434, the method includes thermally disinfecting the drained integrated heat exchanger 110/110' using the integrated thermal disinfection system 140. Where, the thermal disinfection system 140 surrounds the integrated heat exchanger 110/110' and provides heat to disinfect the integrated heat exchanger 110/110'. In embodiments, the drained or emptied integrated heat exchanger 110/110' is hot disinfected at a temperature, such as 95 C, for a specified time to sterilize the integrated heat exchanger 110/110', which includes the prevention of bacterial growth.

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features. Accordingly, the scope of the present disclosure is intended to embrace all such alternatives, modifications, and variations as fall within the scope of the claims, together with all equivalents thereof.

## Claims

1. A system for heating/cooling a target unit, the system comprising:
a heater/cooler unit (102) including:
a heater/cooler (108) that includes a heater/cooler element (114) and a heater/cooler pump (116); and
a heat exchanger (110) that includes a heat exchange element (118), wherein the heater/cooler pump (116) pumps a first fluid through the heater/cooler element (114) and the heat exchange element (118) and back to the heater/cooler pump (116); and
a heat transfer fluid module (104) including a heat transfer fluid tank (120, 300) that defines a heat transfer fluid reservoir (122, 302) configured to contain a second fluid, and a reversible pump (124); and
a target unit (106).

2. The system of claim 1, wherein the reversible pump (124) is configured to drain the second fluid from the heat exchanger (110) and/or the target unit (106) and returned to the heat transfer fluid tank (120).

3. The system of any one of the preceding claims, wherein the heat transfer fluid tank (120, 300) includes a fluid return tube (330) and an internal fluid pick-up tube (332) extending into the fluid heat transfer fluid reservoir (122, 302).

4. The system of claim 3, wherein the heat transfer fluid tank (120, 300) is a rectangular box with a top (320) and a bottom (310), wherein the reversible pump (124) is secured to the top (320).

5. The system of claim 4, wherein the heat transfer fluid pump (304) is fluidly coupled to the internal fluid pick-up tube (332) extending into the heat transfer fluid reservoir (302).

6. The system of claim 5, wherein the internal fluid pick-up tube (332) extends to a bottom portion of the heat transfer fluid reservoir (302).

7. The system of any one of claims 1-6, wherein the heat transfer fluid module is a disposable heat transfer fluid module.

8. The system of claim 7, wherein the heat transfer fluid module (104) includes a transponder (308) configured to limit a number of uses of the heat transfer fluid module (104).

9. The system of any one of claims 1-8, wherein the heater/cooler pump, the heater/cooler element, and the heat exchange element are a closed circuit containing the first fluid.

10. The system of any one of claims 1-9, further comprising an integrated thermal disinfection system (140) around the heat exchanger (110) to provide heat to disinfect the heat exchanger.

11. The system of claim 10, wherein the heat exchanger is configured to be emptied of the second fluid and hot disinfected by the thermal disinfection system (140) at a higher temperature for a period to sterilize the heat exchanger and prevent bacterial growth.

12. A system for heating/cooling a target unit, the system comprising:
a heater/cooler unit (102) configured to regulate temperature of a first fluid and pump the first fluid through a heat exchanger in a closed circuit that prevents contamination due to the first fluid; and
a heat transfer fluid module (104) including a heat transfer fluid tank (120, 300) including a body (318) and a top (320) sealed to the body, the heat transfer fluid tank defining a fluid reservoir (122), the heat transfer fluid module including a reversible pump (124, 304) that is configured to pump a second fluid from the fluid reservoir (122, 302) to the heat exchanger and a target unit (106) and back to the fluid reservoir to regulate temperature of the second fluid and the target unit.

13. The system of claim 12, wherein the reversible pump (124, 304) is configured to drain the second fluid from the heat exchanger (110) and/or the target unit (106) and returned to the heat transfer fluid tank (120).

14. The system of claim 13, wherein the heat transfer fluid tank (120, 300) includes a fluid return tube (330) and an internal fluid pick-up tube (332) extending into the fluid reservoir.

15. The system of claim 14, wherein the heat transfer fluid tank (120, 300) is a rectangular box with a top (320) and a bottom (310), wherein the reversible pump (124) is secured to the top (320).
